# EUROPÄISCHE PATENTSCHRIFT

(11) **EP 0 617 013 B1**
(45) Veröffentlichungstag und Bekanntmachung des Hinweises auf die Patenterteilung: **10.01.1996**
(21) Anmeldenummer: 94103957.0
(22) Anmeldetag: 15.03.1994
(51) Int. Cl.: C07C 271/64

(54) **Copolymerisierbare Oximether und diese enthaltende Copolymerisate**
Copolymerisable oximethers and copolymers containing these
Oxyméthers copolymérisables et copolymères les contenant

(30) Priorität: 22.03.1993 DE 4309193
(43) Veröffentlichungstag der Anmeldung: 28.09.1994
(73) Patentinhaber: BASF Aktiengesellschaft, D-67063 Ludwigshafen (DE)
(72) Erfinder: Bott, Kaspar, Dr., D-68165 Mannheim (DE); Bauer, Gerhard, Dr., D-69469 Weinheim (DE); Haeberle, Karl, Dr., D-67346 Speyer (DE)

(56) Entgegenhaltungen:
- EP-A- 0 177 122
- CHEMICAL ABSTRACTS, vol. 95, no. 17, 26. Oktober 1981, Columbus, Ohio, US; abstract no. 150085g, MOSZCZYNSKI, W. 'Pesticidal activity of novel O-substituted N-alkylphenylcarbamates'
- DATABASE WPI Derwent Publications Ltd., London, GB; AN 91047272 & JP-A-2 311 453 (NIPPON SHOKUBAI K.K.)

## Beschreibung

Die Erfindung betrifft copolymerisierbare Oximether der allgemeinen Formel 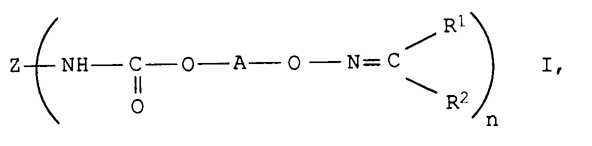
worin A für ein zweiwertiges Bindeglied steht, R¹ und R² unabhängig voneinander für einen C₁- bis C₁₀-Alkyl-, einen C₁-C₁₀-Alkoxy-, einen C₅- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder Alkoxygruppen substituiert sein können, stehen, R¹ oder R² für ein Wasserstoffatom stehen können oder R¹ und R² gemeinsam eine Brücke aus 3 bis 14 Kohlenstoffatomen bilden, wobei ein Teil der Kohlenstoffatome auch Bestandteil eines aromatischen Rings sein kann, Z für einen n-wertigen organischen Rest steht, der eine copolymerisierbare ethylenisch ungesättigte Gruppe enthält, und n eine ganze Zahl von 1 bis 3 ist, und eine Verbindung mit gelichzeitig Z=allyl, A=ortho-phenyloxycarbonyl, R¹=CH₃ und R²=CH₃ ausgenommen ist.

Weiterhin betrifft die Erfindung Copolymerisate, welche die Oximether und auch den oben ausgenommenen Oximether enthalten, sowie ein Verfahren zur Herstellung der Oximether.

Aus JP-A-2311453 und EP-A-177 122 sind Comonomere bekannt, welche Imingruppen enthalten. Es handelt sich jedoch nicht um Oximether.

Bei Copolymerisaten, welche in Beschichtungsmitteln z.B. Lackierungen oder Klebstoffen Verwendung finden, handelt es sich vielfach um vernetzungsfähige Copolymerisate. Durch eine Vernetzung können z.B. Beschichtungen oder Klebstoffbeschichtungen mit guten elastischen Eigenschaften, hoher Kohäsion, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert. Mögliche Vernetzungsmittel sind z.B. Polyisocyanate, welche mit Hydroxylgruppen oder Aminogruppen reagieren.

Aus der DE-A-35 21 618 sind entsprechende wäßrige Klebstoffzubereitungen bekannt, bei denen in Wasser dispergierte Polyisocyanate wäßrigen Dispersionen radikalisch polymerisierter Copolymerisate als Vernetzungsmittel zugesetzt werden. Ähnliche Klebstoffzubereitungen sind auch in der US-A-43 96 738 und DE-A-31 12 117 beschrieben.

Nachteilig bei diesen wäßrigen Zubereitungen ist jedoch die mangelnde Lagerstabilität. Das Polyisocyanat darf daher erst kurz vor seiner Verwendung als Vernetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyante hydrolysieren in wäßriger Dispersion nur noch in untergeordnetem Ausmaß.

Gegenstand der DE-A-38 07 555 ist ein solches, mit Oximen blockiertes Diisocyanat, welches in Wasser dispergiert wird und sich als Zusatz für in Wasser dispergierte Polymerisate eignet.

Vernetzungsreaktionen treten jedoch erst nach Abspaltung des Blockierungsmittels bei Temperaturen ab ca. 130°C auf.

Bisher bekannte wäßrige Klebstoffzubereitungen mit Polyisocyanaten als Vernetzungsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wäßrige Dispersionen sind aus der EP-A-3516 bekannt. Diese Dispersionen enthalten Polyhydrazide, welche mit im Copolymerisat einpolymerisieren Monomeren mit Carbonylgruppen reagieren.

Des weiteren sind aus der EP-A-516 074 Dispersionen bekannt, welche Aminoxyvernetzer enthalten. In den deutschen Patentanmeldungen DE-A-41 21 946 bzw. P 42 19 385.0 sind mit Oxim blockierte Polyisocyanate bzw. copolymerisierbare Oximether als Vernetzungsmittel bekannt. Eine Vernetzung tritt jeweils ein mit Carbonylgruppen enthaltenden Copolymerisaten.

Es ist grundsätzlich erwünscht, weitere lagerstabile, bei Raumtemperatur vernetzende Dispersionen zu entwickeln, um Alternativen zur Polyhydrazidvernetzung zur Verfügung zu stellen.

Aufgabe der vorliegenden Erfindung waren daher vernetzbare Copolymerisate, welche in Dispersion oder Lösung auch in Gegenwart eines Vernetzungsmittels lagerstabil sind und bei Raumtemperatur vernetzt werden können.

Demgemäß wurden die oben definierten copolymerisierbaren Oximether und ein Verfahren zur Herstellung von Oximethern gefunden.

Des weiteren wurden Copolymerisate, welche die copolymerisierbaren Oximether enthalten und die Verwendung der Copolymerisate als Beschichtungsmittel oder Klebstoff gefunden.

Die Copolymerisate, welche die copolymerisierbaren Oximether enthalten, zeigen eine gute Haftung auf unterschiedlichsten Substraten und vernetzen insbesondere mit Aldehyd- oder Ketogruppen enthaltenden Verbindungen.

Bei der Variablen A in der allgemeinen Formel I handelt es sich bevorzugt um eine lineare oder verzweigte Kohlenwasserstoffkette aus 2 bis 12, insbesondere 2 bis 8 Kohlenstoffatomen, welche gegebenenfalls durch 1 bis 3, insbesondere 1 - 2 nicht benachbarte Schwefel- oder Stickstoffatome, vorzugsweise Sauerstoffatome oder einen C₅-C₁₀-Cycloalkylen- oder einen C₅-C₁₀-Arylenring unterbrochen sein kann. Besonders bevorzugt handelt es sich um eine lineare oder verzweigte Kohlenwasserstoffkette aus 2 bis 8 Kohlenstoffatomen.

R¹ und R² stehen bevorzugt unabhängig voneinander für ein Wasserstoffatom, eine C₁₋ bis C₆-Alkylgruppe, C₁- bis C₆-Alkoxygruppe oder einen C₅-C₁₀-Arylrest, insbesondere einen Phenylring. Im Falle des Wasserstoffatoms kann nur einer der beiden Reste R¹ und R² ein Wasserstoffatom sein. n steht für eine ganze Zahl von 1 bis 3, vorzugsweise steht n für 1.

Z steht für einen organischen Rest, der eine copolymerisierbare ethylenisch ungesättigte Gruppe enthält.

Bei Z kann es sich um als solche bekannte Monomere für die radikalische Polymerisation handeln, welche gemäß Formel I durch eine Gruppe 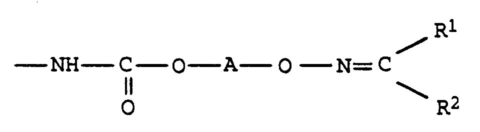
substituiert sind.

Als Z in Betracht kommen z.B. vinylaromatische Reste mit bis zu 20 C-Atomen, (Meth)acrylsäureesterreste der Formel 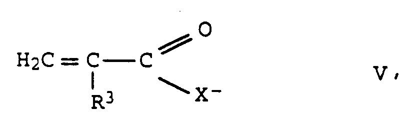
in der R³ ein Wasserstoffatom oder eine Methylgruppe bedeutet und X für ein organisches Bindeglied mit vorzugsweise 1 bis 20 C-Atomen steht. Besonders bevorzugt steht X für eine C₁-C₁₀-Alkylengruppe.

Als Z in Betracht kommen auch ethylenisch ungesättigte Gruppen mit einer Urethangruppe, wie sie durch Umsetzung von gegenüber Isocyanat reaktiven Monomeren mit Isocyanaten insbesondere Diisocyanaten erhältlich sind.

Besonders bevorzugt handelt es sich bei Z um eine (Meth)acroylgruppe der Formel 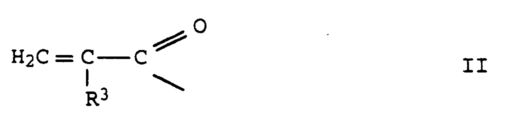
Die Herstellung der copolymerisierbaren Oximether der Formel I kann durch Umsetzung von Oximetheralkoholen der Formel 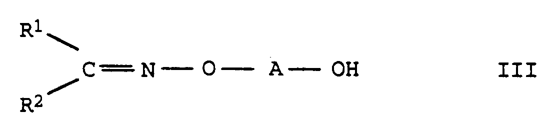
mit einer Isocyanatverbindung der Formel 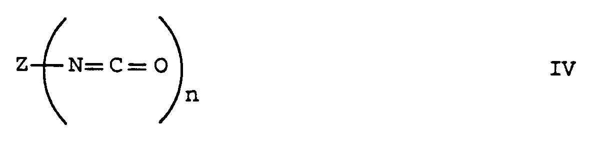
erfolgen.

Die Umsetzung kann in einfacher Weise bei bevorzugt 0 bis 50, insbesondere 0 bis 20°C durch Zusammengeben der Ausgangsverbindungen, vorzugsweise im stöchiometrischen Verhältnis der Oximetheralkohole zu den Isocyanatgruppen erfolgen. Bevorzugt wird die Umsetzung in Gegenwart eines Lösungsmittels vorgenommen. Als Lösungsmittel zu nennen sind z.B. aromatische oder aliphatische Kohlenwasserstoffe, sowie Chlorkohlenwasserstoffe. In der deutschen Patentanmeldung P 42 19 385.0 war bereits ein Verfahren zur Herstellung von copolymerisierbaren Oximethern durch Umsetzung von Oximetheralkoholen mit (Meth)acrylsäurechlorid oder (Meth)acrylsäureanhydrid in Gegenwart einer Base beschrieben. Dieses Verfahren ist mit einem erheblichen Salzanfall verbunden, was jedoch bei dem oben beschriebenen, neuen Verfahren vermieden wird.

Die Oximetheralkohole der Formel III als Ausgangsverbindungen für die Umsetzung sind nach bekannten Verfahren, z.B. durch Umsetzung von Oximen mit Alkylenoxiden wie Ethylenoxid, Propylenoxid etc. oder mit Halogenalkoholen in Gegenwart einer Base erhältlich.

Bei den Isocyanatverbindungen IV handelt es sich um radikalisch polymerisierbare Monomere, also Verbindungen mit einer copolymerisierbaren ethylenisch ungesättigten Gruppe, welche mindestens eine Isocyanatgruppe enthalten.

Als Isocyanatverbindungen in Betracht kommen z.B. (Meth)acryloylisocyanat, C₁-C₁₀-Alkyl-(meth)acrylate, die im Alkylrest durch mindestens eine, vorzugsweise eine Isocyanatgruppe substituiert sind, z.B. 2-Isocyanatoethyl(meth)acrylat, m- oder p-Isopropenyl-α-α'dimethylbenzylisocyanat.

Die Isocyanatverbindungen IV können auch in einfacher Weise hergestellt werden, indem man zunächst Polyisocyanate, insbesondere Diisocyanate, mit ethylenisch ungesättigten Verbindungen umsetzt, so daß mindestens eine freie Isocyanatgruppe verbleibt. Als ethylenisch ungesättigte Verbindungen eignen sich solche, die mindestens eine gegenüber Isocyanat reaktive Gruppe, z.B. eine primäre oder sekundäre Aminogruppe oder vorzugsweise eine Hydroxylgruppe, aufweisen. Diese können in bekannter Weise mit einem Polyisocyanat unter Harnstoff- bzw. Urethanbildung umgesetzt werden. Vorzugsweise werden ethylenisch ungesättigte Verbindungen mit einer Hydroxygruppe, z.B. Hydroxy-C₂-C₁₀-alkyl(meth)acrylate, mit Polyisocyanaten, insbesondere Diisocyanaten, umgesetzt. Geeignete Diisocyanate sind z.B. solche mit der allgemeinen Formel X(NCO)₂, wobei X für einen aliphatischen Kohlenwasserstoffrest mit 4 bis 12 C-Atomen, einen cycloaliphatischen mit 6 bis 15 Atomen oder einen aromatischen oder alkaromatischen Kohlenwasserstoffrest mit 6 bis 15 C-Atomen steht. Genannt seien z.B. 1,4-Butandiisocyanat, 1,6-Hexandiisoyanat, 2,2,4- und 2,4,4-Trimethylhexamethylendiisocyanat, Cyclohexandiisocyanat, Methylcyclohexandiisocyanat, Isophorondiisocyanat, 4,4'-Diisocyanatodiphenylmethan, 4,4'-Diisocyanatodicyclohexylmethan, 2,4- und 2,6-Toluoldiisocyanat.

Die copolymerisierbaren Oximether (im folgenden auch als Monomere a) bezeichnet) können nach üblichen Verfahren der radikalischen Polymerisation mit ethylenisch ungesättigten Monomeren copolymerisiert werden.

Für eine ausreichende Vernetzungsfähigkeit und gute Haftung der erhaltenen Copolymerisate sollte der Gehalt an einpolymerisierten Oximethern a) mindestens 0,01 Gew.-% betragen. Ein Gehalt über 30 Gew.-% ist im allgemeinen nicht notwendig.

Vorzugsweise ist der Gehalt an einpolymerisierten Oximethern im Copolymerisat 0,1 bis 10, besonders bevorzugt 0,1 bis 5 Gew.-%.

Die Copolymerisate enthalten als Hauptmonomere b) 30 - 99,99, vorzugsweise 70 bis 99,9, besonders bevorzugt 85 bis 99,9 Gew.-% eines Monomeren ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigte Nitrile, Vinylhalogenide und nichtaromatischen Kohlenwasserstoffen mit mindestens 2 konjugierten Doppelbindungen.

Als Hauptmonomere zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinvlpropionat und Vinylacetat.

Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und mindestens zwei olefinischen Doppelbindungen seien Butadien, Isopren, und Chloropren genannt.

Die Hauptmonomeren werden auch vorzugsweise im Gemisch eingesetzt.

Die Copolymerisate können weiterhin auch Monomere mit mindestens einer Aldehyd- oder Ketogruppe (Monomere c)) enthalten.

Vorzugsweise handelt es sich um Monomere mit ein oder zwei Aldehyd-, bzw. Ketogruppen oder einer Aldehyd- und einer Ketogruppe und einer radikalisch polymerisierbaren olefinischen Doppelbindung.

In Betracht kommen z.B. Acrolein, Methacrolein, Vinylalkylketone mit 1 bis 20, vorzugsweise 1 bis 10 Kohlenstoffatomen im Alkylrest, Formylstyrol, (Meth-)acrylsäurealkylester mit ein oder zwei Keto- oder Aldehyd-, bzw. einer Aldehyd- und einer Ketogruppe im Alkylrest, wobei der Alkylrest vorzugsweise insgesamt 3 bis 10 Kohlenstoffatome umfaßt, z.B. (Meth)acroyloxalkylpropanale, wie sie in der DE-A-27 22 097 beschrieben sind. Des weiteren eignen sich auch N-Oxoalkyl(meth)acrylamide wie sie z.B. aus der US-A-4 226 007, der DE-A-20 61 213 oder DE-A-22 07 209 bekannt sind.

Besonders bevorzugt sind Acetoacetyl(meth)acrylat, Acetoacetoxyethyl(meth)acrylat und insbesondere Diacetonacrylamid.

Der Gehalt dieser Monomeren beträgt im allgemeinen zwischen 0 und 30 Gew.-%, insbesondere zwischen 0 und 10, besonders bevorzugt zwischen 0 und 5 Gew.-%.

Das Copolymerisat kann selbst- oder fremdvernetzbar sein. Im Falle der Selbstvernetzbarkeit enthält es sowohl copolymerisierbare Oximether als auch vorzugsweise Monomere mit mindestens einer Keto- oder Aldehydgruppe. Die Vernetzung des Copolymerisats tritt dann ohne Zusatz eines Vernetzungsmittels durch Reaktion der Oximgruppe mit den Keto- oder Aldehydgruppe im gleichen Copolymerisat ein.

Der Gehalt am Monomer mit mindestens einer Keto- oder Aldehydgruppe c) im Copolymerisat sollte dann vorzugsweise mindestens 0,1 Gew.-% betragen. Die maximal mögliche Menge des Hauptmonomeren reduziert sich dann um 0,1 Gew.-%. Für eine gute Haftung ist ein Gehalt an Monomeren c) nicht notwendig.

Als weitere von den Monomeren a) bis c) verschiedene Monomere d), welche im Copolymerisat enthalten sein können, sind z.B. Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, wie 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder Cycloalkylester, wie Cyclohexyl(meth)acrylat, Phenylethyl-(meth)acrylat, Phenylpropyl(meth)acrylat oder Acrylsäureester von heterocyclischen Alkoholen wie Furfuryl(meth)acrylat genannt.

Darüber hinaus kommen noch weitere Monomere wie (Meth)acrylamid, sowie deren am Stickstoff mit C₁-C₄-Alkyl substituierten Derivate, in Betracht.

Von Bedeutung sind auch hydroxyfunktionelle Monomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₂-C₈-hydroxyalkylester, wie n-Hydroxyethyl-, n-Hydroxypropyl- oder n-Hydroxybutyl(meth)acrylat.

Die Mitverwendung von Comonomeren mit salzbildenden Gruppen empfiehlt sich für die Herstellung selbstdispergierbarer Copolymerisate, die z.B. für wäßrige Sekundärdispersionen geeignet sind. Monomere mit salzbildenden Gruppen sind insbesondere Itaconsäure, Acrylsäure und Methacrylsäure.

Der Gewichtsanteil der weiteren Comonomeren im Copolymerisat kann 0 bis 50 %, vorzugsweise 0 bis 20 % und ganz besonders bevorzugt 0 bis 10 Gew.-% betragen.

Die Herstellung des Copolymerisats A) erfolgt durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid mit Fettalkoholen, -säuren oder Phenol, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak zu Carbonsäuregruppen enthaltenden Copolymerisaten, in Wasser ohne Verwendung eines Emulgators oder Dispergierhilfsmittels dispergiert. Das organische Lösungsmittel kann abdestilliert werden. Die Herstellung von wäßrigen Sekundärdispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan.

Die Art und Menge der Comonomeren wird zweckdienlicherweise so gewählt, daß das erhaltene Copolymerisat eine Glasübergangstemperatur zwischen bevorzugt -60 und +140°C, besonders bevorzugt -30 und +80°C, z.B. für eine Verwendung als Lackbindemittel und ganz besonders bevorzugt, vor allem im Falle der Verwendung als Klebstoff, zwischen -30 und +20°C hat. Die Glasübergangstemperatur des Copolymerisats läßt sich nach üblichen Methoden wie Differentialthermoanalyse oder Differential Scanning Calorimetrie (s. z.B. ASTM 3418/82, sog. "midpoint temperature") bestimmen.

Im Falle, daß das Copolymerisat nicht selbstvernetzend ist, also keine Monomere c) enthält, kann dem Copolymerisat geeigneterweise für die Vernetzung ein Vernetzungsmittel zugegeben werden. Bei dem Vernetzungsmittel handelt es sich üblicherweise um eine Verbindung, welche mindestens zwei Keto- oder Aldehydgruppen, bzw. mindestens eine Keto- und eine Aldehydgruppe enthält.

Solche Verbindungen sind z.B. Succindialdehyd, Glutardialdehyd oder Terephthaldialdehyd.

Geeignet als Vernetzungsmittel sind insbesondere auch radikalische Copolymerisate - im folgenden auch polymeres Vernetzungsmittel genannt - welche voranstehend genannte Monomere c) einpolymerisiert enthalten.

In Betracht kommen z.B. polymere Vernetzungsmittel, die aus 30 - 99,9 Gew.-%, vorzugsweise 70 - 99,9 Gew.-% der Monomeren b), 0,1 - 30 Gew.-%, vorzugsweise 0,1 - 10 Gew.-% der Monomeren c) und 0 - 50 Gew.-%, vorzugsweise 0 - 20 Gew.-% der Monomeren d) aufgebaut sind. Über die Art der Monomeren, die Glasübergangstemperatur und die Herstellung gilt vorzugsweise das voranstehend über obige Copolymerisate gesagte.

Des weiteren können die erfindungsgemäßen Copolymerisate auch mit Hydroxylgruppen enthaltenden Verbindungen, insbesondere auch Hydroxylgruppen-enthaltenden Copolymerisaten vernetzen.

Das Vernetzungsmittel, soweit gewünscht, wird vorzugsweise zur Lösung oder Dispersion der Copolymerisate gegeben.

Es ist jedoch auch möglich, das Copolymerisat und das Vernetzungsmittel erst bei der Anwendung, z.B. der Beschichtung von Oberflächen zusammenzubringen. Dazu könnte auf die Oberfläche z.B. zunächst das Vernetzungsmittel als sog. Primer aufgebracht werden und anschließend die Beschichtung mit der Dispersion oder Lösung der Copolymerisate erfolgt.

Die Lösung oder Dispersion der erfindungsgemäßen Copolymerisate eigent sich z.B. für die Verwendung als Anstrich- und Beschichtungsmittel, z.B. als Schutzüberzug oder Lackierungen, auch für dekorative Zwecke, für unterschiedliche Substrate mit Kunststoff-, Holz- oder Metalloberflächen oder z.B. für Textilien, Vliesstoffe, Leder oder Papier. Sie eignen sich ebenfalls für Anwendungen in der Bauchemie z.B. als Klebstoffe, Dichtungsmassen, Bindemittel oder ähnliches.

Die Dispersionen der Lösungen können je nach Verwendungszweck noch übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Auch Fungizide zur Konservierung können zugesetzt werden. Diese kommen im allgemeinen in Mengen von 0,02 bis 1 Gew.-%, bezogen auf die Dispersionen oder Lösungen zum Einsatz. Geeignete Fungizide sind beispielsweise Phenol- oder Kresol-Derivate oder zinnorganische Verbindungen.

Bei einer Verwendung für Lackierungen können die erfindungsgemäßen Dispersionen oder Lösungen lacktypische Zusätze wie Filmbildehilfsmittel, Pigmente, Mattierungsmittel, Verdicker, Pigmentverteiler, Entschäumer etc., sowie natürliche oder synthetische harze, z.B. Alkydharze oder Polyurethane enthalten.

Besonders eignen sich die Dispersionen oder Lösungen auch als Dichtstoff- oder Klebstoffzubereitungen, insbesondere als Kaschierklebstoff zur Herstellung von Verbundfolien und Glanzfolien. Als solche können sie neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Die Dispersionen oder Lösungen der selbstvernetzenden oder fremdvernetzenden Copolymerisate, welche noch ein Vernetzungsmittel enthalten, sind lagerstabil. Die Vernetzung tritt bereits bei Raumtemperatur mit Verflüchtigung des Lösungsmittels ein. Durch die Gegenwart von Metallsalzen werden die Vernetzungsfähigkeit und die guten Haftungseigenschaften der Copolymerisate nicht beeinträchtigt.

Die mit diesen Dispersionen oder Lösungen hergestellten Beschichtungen oder Verklebungen weisen eine gute Chemikalien- oder Lösungsmittelbeständigkeit und eine gute innere Festigkeit (Kohäsion) auf.

### Herstellung von copolymerisierbaren Oximethern V1 - V5

### (3-Methacrylamido-carbonyloxypropyl)-acetonoximether (V1)

Eine Lösung von 55,5 g (0,50 mol) Methacroyl-isocyanat in 50 ml Dichlormethan wurde innerhalb von 30 min bei 10°C in eine Mischung aus 65,5 g (0,50 mol) O-(3-Hydroxypropyl)-acetonoxim und 100 ml Dichlormethan eingetropft. Man ließ 2 h bei 10°C nachreagieren und verdampfte das Lösungsmittel bei Raumtemperatur im Vakuum. Der Destillationsrückstand bestand aus 121 g (Ausbeute 100 %) kristallisiertem Produkt, das nach Umkristallisation aus Methyltert.-butylether (bei -25°C) bei 51 - 53°C schmolz.

| Elementaranalyse: | | | | |
|---|---|---|---|---|
| Ber. | C 54,53 | H 7,49 | O 26,41 | N 11,56 |
| | C 54,7 | H 7,6 | O 26,6 | N 11,8 |

Für alle copolymerisierbaren Oximether der Tabelle 1 konnten korrekte Elementaranalysen und H-NMR-Spektren erhalten werden.

Die Herstellung der copolymerisierbaren Oximether V2 bis V5 wurde analog V1 durchgeführt. In Tabelle 1 sind die Einsatzstoffe und Produkte mit ihren Strukturformeln angegeben.

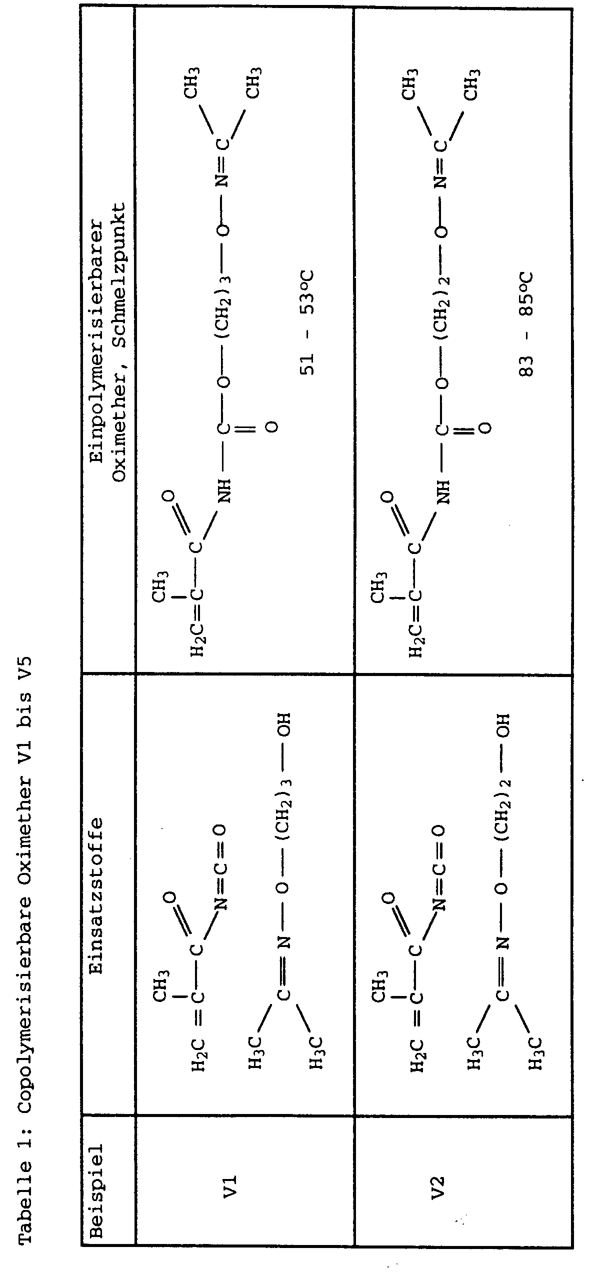

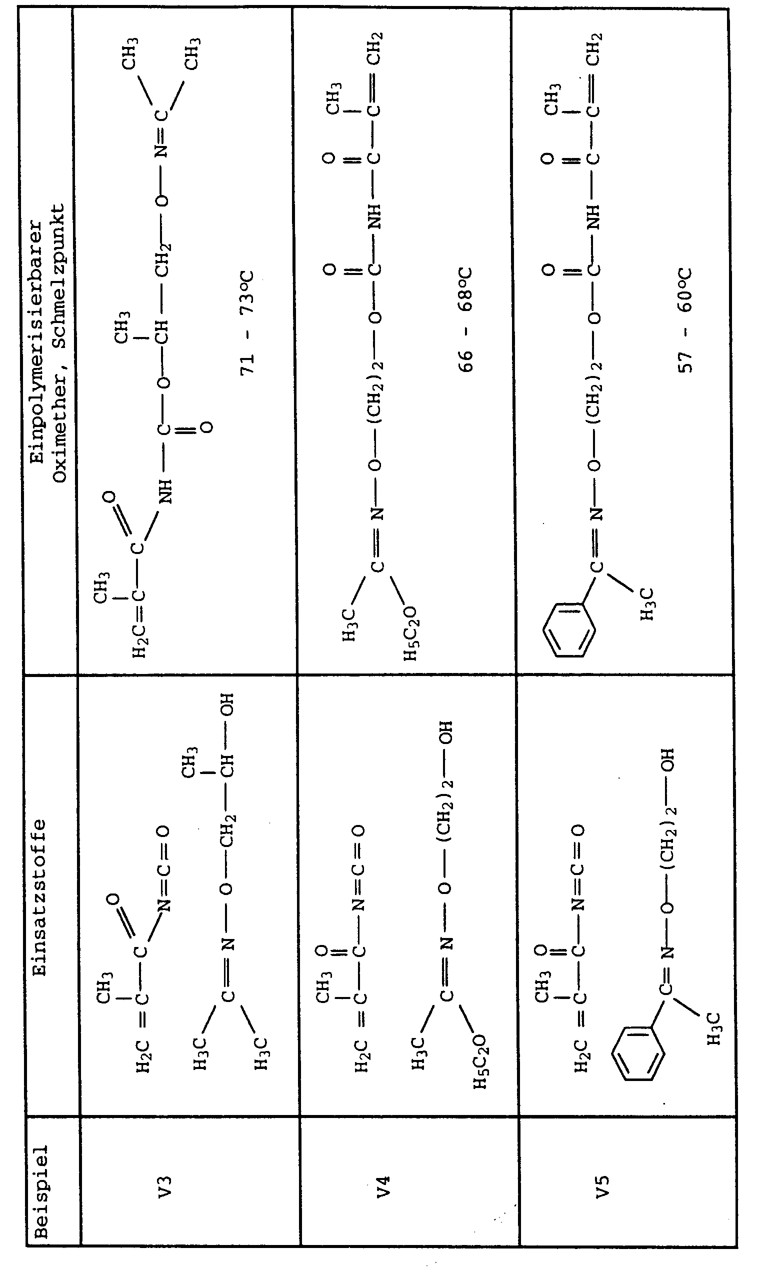

### Herstellung der Dispersionen D1 bis D15

### Dispersion D1

In einem Reaktionsgefäß mit Rührer und zwei Zulaufgefäßen (Zulauf 1 und Zulauf 2) wurden 200 g entsalztes Wasser, 37 g des Zulaufs 1 (siehe unten) und 20 g des Zulaufs 2 vorgelegt und auf 85°C erwärmt. Nach 15 Minuten wurde innerhalb von 2 h gleichmäßig der Zulauf 1 sowie innerhalb von 2,5 h gleichmäßig der Zulauf 2 zum Reaktionsgefäß zugegeben. Nach der letzten Initiator-Zugabe (Zulauf 2) wurde die Dispersion noch 1 Stunde bei 85°C gerührt.

### Zulauf 1: (dieser Zulauf wird während der Polymerisation gerührt)

107,5 g entsalztes Wasser
400 g Ethylacrylat
90 g Methylmethacrylat
50 g 20 gew.-%ige wäßrige Diacetonacrylamid-Lösung
50 g 20 gew.-%ige Lösung des Natriumsalzes von p-Dodecyldiphenyletherdisulfonat in Wasser (Emulgator)
50 g 20 gew.-%ige Lösung des Umsetzungsprodukts von p-Isononylphenol mit ca. 50 Mol Ethylenoxid in Wasser (Emulgator)

### Zulauf 2:

100 g entsalztes Wasser
3 g Natriumpersulfat
In entsprechender Weise wurden die Dispersionen D2 bis D15 hergestellt. Die Zusammensetzung der jeweiligen Copolymerisate ist in Tabelle 2 angegeben.

### Vernetzbarkeit (Prüfung durch Quellungsverhalten und Bestimmung der extrahierbaren Anteile)

Je 10 g der Dispersionen wurden verfilmt und die Filme 1 Woche bei Raumtemperatur getrocknet. Anschließend wurde das Quellungsverhalten als Maß für den Vernetzungsgrad dieser Filme in Tetrahydrofuran untersucht, indem ca. 1 g der verfilmten Proben in Tetrahydrofuran 2 Tage gelagert und die Lösungsmittelaufnahme in % gemessen wurde (Ergebnisse in Tab. 6).

Bei vernetzten Polymeren kommt es durch Aufnahme von Lösungsmittel zur Quellung. Mit steigendem Vernetzungsgrad wird die Quellung geringer, da weniger Lösungsmittel vom eng vernetzten Polymer aufgenommen werden kann. Nicht oder kaum vernetzte Polymere werden durch Lösungsmittel zum großen Teil gelöst oder quellen bei Vorhandensein einer geringen Anzahl von Vernetzungsstellen übermäßig stark an.

Die extrahierbaren Anteile wurden bestimmt durch Rückwiegen bei Raumtemperatur nach Trocknung im Trockenschrank bei 80°C während 4h.

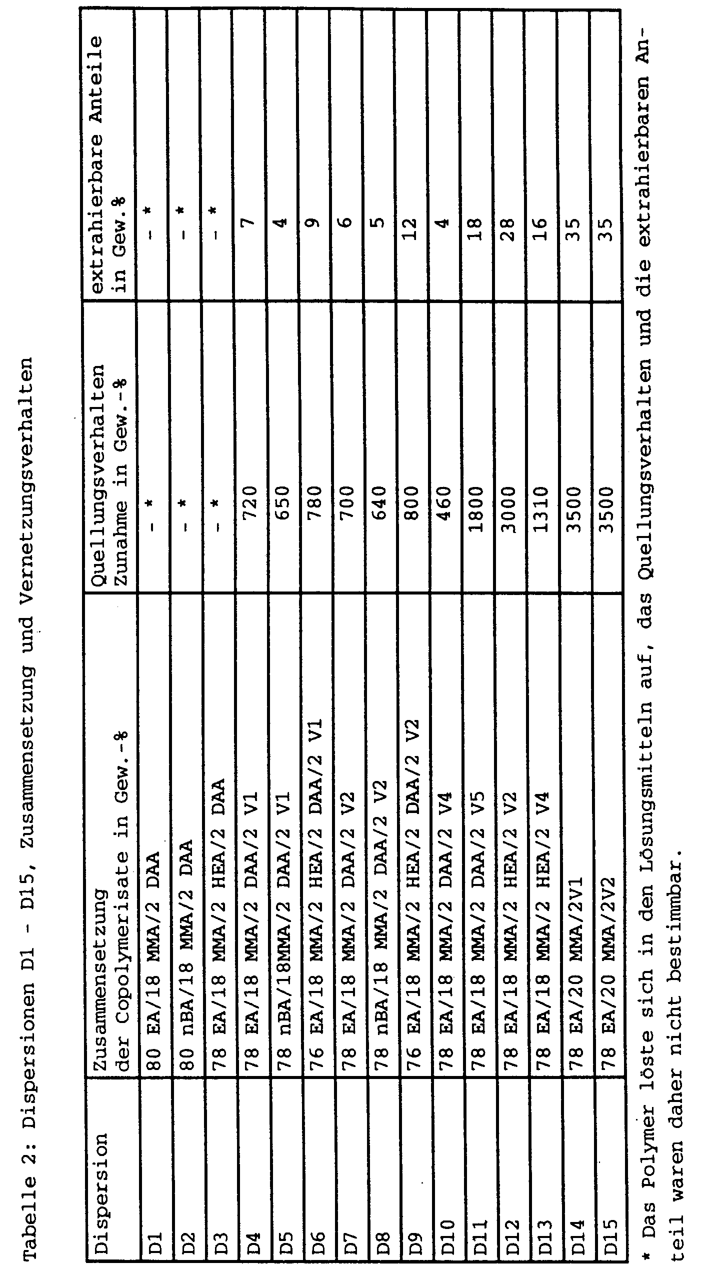

### Abkürzungen

- EA: Ethylacrylat
- nBA: n Butylacrylat
- MMA: Methylmethacrylat
- HEA: Hydroxyethylacrylat
- DAA: Diacetonacrylamid
Bei den Dispersionen D1 bis D3 tritt keine Vernetzung ein. Die selbstvernetzenden Dispersionen D4 bis D11 enthalten einen Vernetzer V1 bis V5 und ein Monomer mit einer Ketogruppe. Sie sind schon bei Raumtemperatur gut vernetzbar. Die Ergebnisse der Dispersionen D12 bis D15 zeigen, daß die einpolymerisierten Oximether auch mit Hydroxylgruppen (D12, D13) und mit sich selbst (D14, D15) vernetzen können.

### Beispiele für Holzlacke

### Holzlack 1

### Vorlage:

80.00 g Natriumlaurylsulfat (10%ig in H₂O)
59.90 g Zulauf 1
30.77 g Zulauf 2
270.00 g H₂O

### Zulauf 1:

80.00 g Natriumlaurylsulfat (10%ig in H₂O)
60.00 g DAAM (20%ig in H₂O)
24.00 g Acrylsäure
12.00 g Divinylbenzol
738.80 g Butylmethacrylat
13.20 g copolym. Oximether V1
270.00 g H₂O

### Zulauf 2:

4.00 g Natriumpersulfat
303.69 g H₂O

### Zulauf 3:

7.36 g Ammoniak (25%ig H₂O)
Die Vorlage wurde auf 85°C aufgeheizt und 15 Minuten anpolymerisiert. Zulauf 1 wurde im Verlauf von 2 h, Zulauf 2 in 2,5 h zugefahren.

Nach dem Abkühlen wurde mit Zulauf 3 pH 7 eingestellt.

| | |
|---|---|
| Quellung in Tetrahydrofuran | 580 Gew.-% |
| extrahierbare Anteile | 5 Gew.-% |

### Holzlack 2

### Vorlage:

80.00 g Natriumlaurylsulfat (10%ig in H₂O)
58.70 g Zulauf 1
30.77 g Zulauf 2
294.00 g H₂O

### Zulauf 1:

80.00 g Natriumlaurylsulfat (10%ig in H₂O)
20.00 g AAEM (20%ig in H₂O)
24.00 g Acrylsäure
12.00 g Divinylbenzol
726.40 g Butylmethacrylat
17.60 g copolym. Oximether V1
294.00 g H₂O

### Zulauf 2:

4.00 g Natriumpersulfat
303.69 g H₂O

### Zulauf 3:

7.36 g Ammoniak (25%ig in H₂O)
Die Herstellung erfolgt analog Holzlack 1.

| | |
|---|---|
| Quellung in Tetrahydrofuran | 490 Gew.-% |
| extrahierbare Anteile | 4 Gew.-% |

## Patentansprüche

1. Copolymerisierbare Oximether der allgemeinen Formel 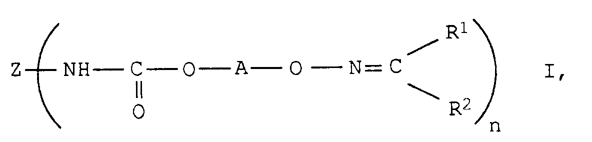 worin A für ein zweiwertiges Bindeglied steht, R¹ und R² unabhängig voneinander für einen C₁- bis C₁₀-Alkyl-, einen C₁-C₁₀-Alkoxy-, einen C₅- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder -Alkoxygruppen substituiert sein können, stehen, R¹ oder R² für ein Wasserstoffatom stehen können oder R¹ und R² gemeinsam eine Brücke aus 3 bis 14 Kohlenstoffatomen bilden, wobei ein Teil der Kohlenstoffatome auch Bestandteil eines aromatischen Rings sein kann, Z für einen n-wertigen organischen Rest steht, der eine copolymerisierbare ethylenisch ungesättigte Gruppe enthält, n eine ganze Zahl von 1 bis 3 ist und eine Verbindung mit gleichzeitig Z=allyl, A=ortho-phenyloxycarbonyl, R¹=CH₃ und R²=CH₃ ausgenommen ist.

2. Copolymerisierbare Oximether gemäß Anspruch 1, wobei Z für eine (Meth)acroylgruppe der Formel 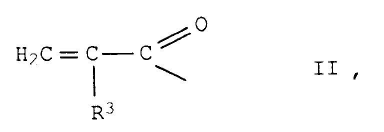 in der R³ ein Wasserstoffatom oder eine Methylgruppe bedeutet, steht und n=1 ist.

3. Verfahren zur Herstellung von copolymerisierbaren Oximethern nach Anspruch 1, dadurch gekennzeichnet, daß man Oximetheralkohole der allgemeinen Formel 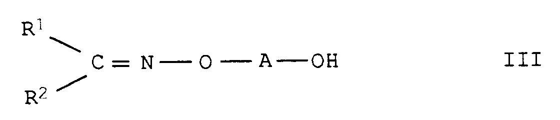 mit einer Isocyanatverbindung der allgemeinen Formel 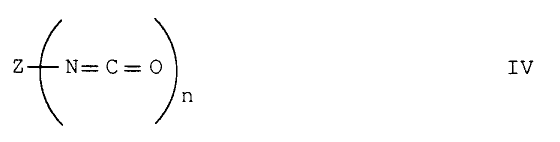 zur Umsetzung bringt.

4. Copolymerisate, enthaltend 0,01 bis 30 Gew.-% der Oximether gemäß Anspruch 1.

5. Copolymerisate nach Anspruch 4, enthaltend
a) 0,01 bis 30 Gew.-% eines Oximethers der Formel I, worin die Reste Z, A, R¹ und R² die in Anspruch 1 angegebene Bedeutung haben und eine Verbindung mit Z=allyl, A=ortho-phenyloxycarbonyl, R¹=CH₃ und R²=CH₃ nicht ausgenommen ist.
b) 30 bis 99,99 Gew.-% mindestens eines C₁ bis C₂₀-Alkyl(meth-)acrylats, eines Vinylesters von 1 bis 20 C-Atome enthaltenden Carbonsäuren, eines Vinylaromaten mit bis zu 20 C-Atomen, eines ethylenisch ungesättigten Nitrils mit 3-6 C-Atomen, eines Vinylhalogenids oder eines nichtaromatischen Kohlenwasserstoffs mit 4 bis 8 C-Atomen und mindestens 2 konjugierten Doppelbindungen,
c) 0 bis 30 Gew.-% mindestens eines Comonomeren mit mindestens einer Keto- oder Aldehydgruppe und
d) 0 bis 50 Gew.-% mindestens eines weiteren Monomeren,
wobei das Copolymerisat eine Glasübergangstemperatur zwischen -60 und +140°C hat.

6. Verwendung von Copolymerisaten gemäß Anspruch 4 oder 5 als Beschichtungsmittel.

7. Verwendung von Copolymerisaten gemäß Anspruch 4 oder 5 als Klebstoffe.

## Claims

1. A copolymerizable oxime ether of the formula 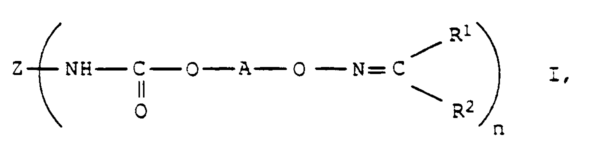 where A is a divalent linking member, R¹ and R² independently of one another are each C₁-C₁₀-alkyl, C₁-C₁₀-alkoxy, C₅-C₁₀-cycloalkyl or C₅-C₁₀-aryl, each of which may furthermore contain 1-3 nonadjacent nitrogen, oxygen or sulfur atoms as heteroatoms in the carbon chain or in the carbon ring and may be substituted by from one to three C₁-C₄-alkyl or C₁-C₄-alkoxy groups, R¹ and R² may be hydrogen or R¹ and R² together form a bridge of 3 to 14 carbon atoms, where some of the carbon atoms may furthermore be part of an aromatic ring, Z is an n-valent organic radical which contains a copolymerizable ethylenically unsaturated group and n is an integer of from 1 to 3, excluding a compound where at one and the same time Z is allyl, A is ortho-phenoxycarbonyl, R¹ is CH₃ and R² is CH₃.

2. A copolymerizable oxime ether as claimed in claim 1, wherein Z is a (meth)acryloyl group of the formula 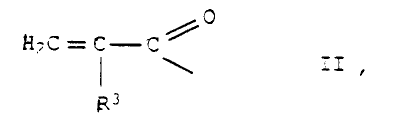 where R³ is hydrogen or methyl and n is 1.

3. A process for the preparation of a copolymerizable oxime ether as claimed in claim 1, wherein an oxime ether alcohol of the formula 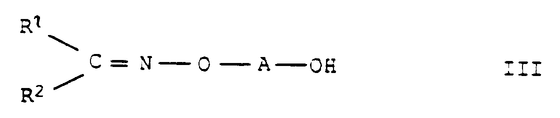 is reacted with an isocyanate compound of the formula

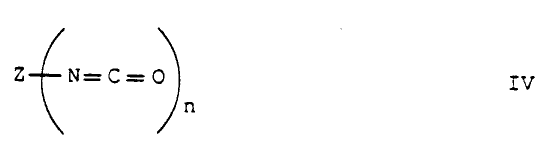

4. A copolymer containing from 0.01 to 30% by weight of an oxime ether as claimed in claim 1.

5. A copolymer as claimed in claim 4, containing
a) from 0.01 to 30% by weight of an oxime ether of the formula I where Z, A, R¹ and R² have the meanings stated in claim 1, but not excluding a compound where Z is allyl, A is ortho-phenoxycarbonyl, R¹ is CH₃ and R² is CH₃,
b) from 30 to 99.99% by weight of at least one C₁-C₂₀-alkyl (meth)acrylate, one vinyl ether with carboxylic acids of 1 to 20 carbon atoms, one vinyl aromatic of up to 20 carbon atoms, one ethylenically unsaturated nitrile of 3-6 carbon atoms, one vinyl halide or one nonaromatic hydrocarbon having 4 to 8 carbon atoms and at least 2 conjugated double bonds,
c) from 0 to 30% by weight of at least one comonomer having at least one keto or aldehyde group and
d) from 0 to 50% by weight of at least one further monomer,
the copolymer having a glass transition temperature of from -60 to +140°C.

6. Use of a copolymer as claimed in claim 4 or 5 as a coating material.

7. Use of a copolymer as claimed in claim 4 or 5 as an adhesive.

## Revendications

1. Oximéthers copolymérisables répondant à la formule générale 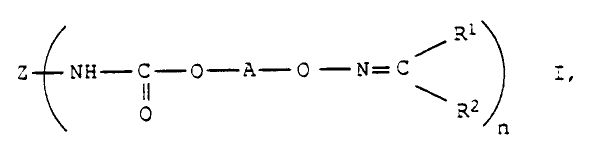 dans laquelle A représente un élément de liaison bivalent, R¹ et R² représentent, chacun indépendamment l'un de l'autre, un radical alkyle en C₁ à C₁₀, alcoxy en C₁ à C₁₀, cycloalkyle en C₅ à C₁₀, ou aryle en C₅ à C₁₀, qui peut aussi contenir de 1 à 3 atomes d'azote, d'oxygène ou de soufre non voisins, à titre d'éthéroatomes, dans la chaîne carbonée ou dans le noyau ou cycle carboné et qui peut être substitué par de 1 à 3 radicaux alkyle ou alcoxy en C₁ à C₄, R¹ ou R² peut représenter un atome d'hydrogène, ou bien R¹ et R² forment ensemble un pont constitué de 3 à 14 atomes de carbone, où une partie des atomes de carbone peuvent également former une partie constituante d'un noyau aromatique, Z représente un radical organique n-valant, qui contient un radical éthyléniquement saturé copolymérisable, n représente un nombre entier dont la valeur varie de 1 à 3 et à l'exclusion d'un composé dans lequel simultanément Z=allyle, A=ortho-phényloxycarbonyle, R¹=CH₃ et R²=CH₃.

2. Oximéthers copolymérisables suivant la revendication 1, dans lesquels Z représente un radical (méth)acryloyle de la formule 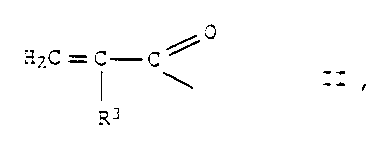 dans laquelle R³ représente un atome d'hydrogène ou le radical méthyle et n est égal à 1.

3. Procédé de préparation d'oximéthers copolymérisables suivant la revendication 1, caractérisé en ce que l'on fait réagir des oximétheralcools de la formule générale 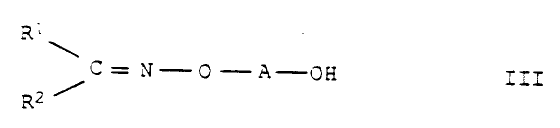 avec un composé d'isocyanate de la formule générale

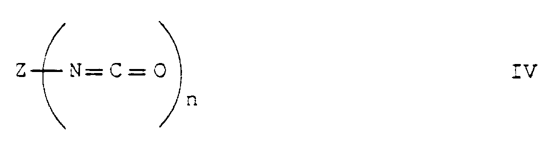

4. Copolymères qui contiennent de 0,01 à 30% en poids des oximéthers suivant la revendication 1.

5. Copolymères suivant la revendication 4, qui contiennent
a) 0,01 à 30% en poids d'un oximéther de la formule I dans laquelle les restes Z, A, R¹ et R² possèdent les significations qui leur ont été attribuées dans la revendication 1, à l'exclusion d'un composé avec Z=allyle, A=ortho-phényloxycarbonyle, R¹=CH₃ et R²=CH₃,
b) 30 à 99,99% en poids d'au moins un (méth)acrylate d'alkyle en C₁ à C₂₀, d'un ester vinylique d'acides carboxyliques contenant de 1 à 20 atomes de carbone, d'un composé vinylaromatique comptant jusqu'à 20 atomes de carbone, d'un nitrile éthyléniquement insaturé comportant de 3 à 6 atomes de carbone, d'un halogénure de vinyle ou d'un hydrocarbure non aromatique comportant de 4 à 8 atomes de carbone et au moins deux doubles liaisons conjuguées,
c) 0 à 30% en poids d'au moins un comonomère avec au moins un radical céto ou aldéhyde et
d) 0 à 50% en poids d'au moins un autre monomère,
où le copolymère possède une température de transition vitreuse comprise entre -60 et +140°C.

6. Utilisation de copolymères suivant la revendication 4 ou 5 à titre d'agents de revêtement.

7. Utilisation de copolymères suivant la revendication 4 ou 5 à titre de colles.
